Europäisches Patentamt

European Patent Office

Office européen des brevets

(19)

(11) **EP 1 530 163 A2**

(12) **EUROPEAN PATENT APPLICATION**

(43) Date of publication:
    **11.05.2005  Bulletin 2005/19**

(51) Int Cl.[7]: **G06T 11/00**

(21) Application number: **04256827.9**

(22) Date of filing: **04.11.2004**

(84) Designated Contracting States:
    **AT BE BG CH CY CZ DE DK EE ES FI FR GB GR
    HU IE IS IT LI LU MC NL PL PT RO SE SI SK TR**
    Designated Extension States:
    **AL HR LT LV MK YU**

(30) Priority:  **04.11.2003  JP 2003373871**

(71) Applicant: **GE Medical Systems Global
    Technology Company LLC
    Waukesha, Wisconsin 53188-1696 (US)**

(72) Inventor: **Hagiwara, Akira
    Hino-shi Tokyo 191-8503 (JP)**

(74) Representative: **Pedder, James Cuthbert
    London Patent Operation,
    General Electric International, Inc.,
    15 John Adam Street
    London WC2N 6LU (GB)**

(54) **CT image producing method and x-ray CT apparatus**

(57)    For the purpose of scanning a periodically moving subject to be imaged using a multi-row detector and producing a CT image at a desired phase with reduced cone-beam artifacts, the periodically moving subject to be imaged is scanned over a plurality of cycles while rotating an X-ray tube (21) and a multi-row detector (24) around the subject to be imaged and rectilinearly moving a table (12) supporting thereon the subject to be imaged; projection data collected in the same phase range in the plurality of cycles are extracted, the projection data being collected at a projection point onto which a pixel on a reconstruction plane is projected on a multi-row detector plane in a direction of X-ray transmission; and a CT image is produced based on the extracted projection data.

FIG. 1

EP 1 530 163 A2

## Description

The present invention relates to a CT image producing method and an X-ray CT (computed tomography) apparatus, and more particularly to a CT image producing method and an X-ray CT apparatus for scanning a periodically moving subject to be imaged using a multi-row detector, and producing a CT image at a desired phase with reduced cone-beam artifacts.

A conventional multi-sector reconstruction technique is known, which involves scanning a heart over a plurality of cardiac cycles, gathering data at a desired cardiac phase from among the obtained data to create a data set needed in image reconstruction, and reconstructing a CT image at the desired cardiac phase from the data set (see Japanese Patent Application Laid Open No. 2003-052688 and "All about Multi-row Detector Helical CT," edited by Mutsumasa TAKAHASHI and Akihiko ARAKAWA, Kanehara & Co., Ltd., (February 1, 2002) pp. 86 — 89 ('Image Reconstruction By Electrocardiographic Synchronization,' written by Seiki HAMADA, Hironobu NAKAMURA, and Hiroaki NAITO)) for example.

In the conventional multi-sector reconstruction technique, it is assumed that an X-ray beam is parallel to a reconstruction plane (slice plane) even if the scan is conducted using a multi-row detector, and data at a desired cardiac phase is gathered from among data of a detector row corresponding to the position of the reconstruction plane (the slice position).

If, however, the reconstruction plane lies at a position away from the center of the multi-row detector (the center being the position straight below the X-ray tube), the X-ray beam is actually not parallel to the reconstruction plane, resulting in cone-beam artifacts in the CT image.

The present invention therefore seeks to provide a CT image producing method and an X-ray CT apparatus for scanning a periodically moving subject to be imaged using a multi-row detector, and producing a CT image at a desired phase with reduced cone-beam artifacts.

In its first aspect, the present invention provides a CT image producing method characterized in comprising: scanning a periodically moving subject to be imaged over a plurality of cycles while making relative rotation of at least one of an X-ray tube and a multi-row detector around said subject to be imaged; extracting projection data collected in the same phase range in said plurality of cycles, said projection data being collected at a projection point onto which a pixel on a reconstruction plane is projected on a multi-row detector plane in a direction of X-ray transmission; and producing a CT image based on said extracted projection data.

As used herein, the term "relative rotation" includes: for a subject to be imaged placed in between the X-ray tube and multi-row detector, rotating at least one of the X-ray tube and multi-row detector around the subject to be imaged without rotating the subject to be imaged; rotating the subject to be imaged around its axis without rotating the X-ray tube and multi-row detector; and rotating the subject to be imaged around its axis and counter-rotating at least one of the X-ray tube and multi-row detector around the subject to be imaged.

According to the CT image producing method of the first aspect, since a multi-sector reconstruction technique is employed, a CT image of a periodically moving subject to be imaged can be produced at a desired phase. Moreover, since the CT image is produced by extracting projection data of a detector row and channel upon which an X-ray beam passing through a pixel on a reconstruction plane impinges, cone-beam artifacts can be reduced.

In its second aspect, the present invention provides a CT image producing method characterized in comprising: scanning a periodically moving subject to be imaged over a plurality of cycles while making relative rotation of at least one of an X-ray tube and a multi-row detector around said subject to be imaged and making relative rectilinear motion of said X-ray tube and multi-row detector with respect to said subject to be imaged; extracting projection data collected in the same phase range in said plurality of cycles, said projection data being collected at a projection point onto which a pixel on a reconstruction plane is projected on a multi-row detector plane in a direction of X-ray transmission; and producing a CT image based on said extracted projection data.

As used herein, the term "relative rectilinear motion" includes: for a subject to be imaged placed in between the X-ray tube and multi-row detector, rectilinearly moving the subject to be imaged (or the table on which the subject to be imaged is laid) without rectilinearly moving the X-ray tube and multi-row detector; rectilinearly moving the X-ray tube and multi-row detector without rectilinearly moving the subject to be imaged (or the table on which the subject to be imaged is laid); and rectilinearly moving the subject to be imaged (or the table on which the subject to be imaged is laid) and rectilinearly moving the X-ray tube and multi-row detector in the opposite direction.

According to the CT image producing method of the second aspect, since a multi-sector reconstruction technique is employed, a CT image of a periodically moving subject to be imaged can be produced at a desired phase. Moreover, since the CT image is produced by extracting projection data of a detector row and channel upon which an X-ray beam passing through a pixel on a reconstruction plane impinges, cone-beam artifacts can be reduced.

In its third aspect, the present invention provides the CT image producing method having the aforementioned configuration, characterized in comprising: when a view range V needed in image reconstruction is divided into N segments from first through N-th segments (N ≥ 2), repeating N times the collection of data for one segment in one cycle.

According to the CT image producing method of the third aspect, the imaging time required is of only a period corresponding to N cycles.

In its fourth aspect, the present invention provides the CT image producing method having the aforementioned configuration, characterized in that: representing the cycle of motion of the subject to be imaged as T, the time width of said phase range as τe, and the cycle of rotation of the X-ray tube and multi-row detector as Ts,

$$Ts = T - \tau e$$

or

$$Ts = T + \tau e$$

is defined.

According to the CT image producing method of the fourth aspect, projection data of an adjacent segment can be collected for each cycle of the subject to be imaged.

In its fifth aspect, the present invention provides the CT image producing method having the aforementioned configuration, characterized in that: the view range V needed in image reconstruction is V = (180° + fan beam angle).

According to the CT image producing method of the fifth aspect, the imaging time can be reduced as compared with a view range V needed in image reconstruction of V = 360°.

In its sixth aspect, the present invention provides the CT image producing method having the aforementioned configuration, characterized in comprising: producing a CT image by a three-dimensional image reconstruction technique.

According to the CT image producing method in the sixth aspect, since image reconstruction is performed according to a three-dimensional image reconstruction technique, artifacts caused by a large cone angle are prevented.

Known three-dimensional image reconstruction techniques include the Feldkamp method and the weighted Feldkamp method.

In its seventh aspect, the present invention provides the X-ray CT imaging method having the aforementioned configuration, characterized in that said three-dimensional image reconstruction technique is a three-dimensional backprojection method comprising: extracting projection data corresponding to a projection line formed by projecting one line or a plurality of parallel lines at spacings of a plurality of pixels on a reconstruction plane onto a multi-row detector plane in a direction of X-ray transmission; generating projection line data by multiplying said extracted projection data by a cone beam reconstruction weight; generating back-projected line data by filtering said projection line data; determining back-projected pixel data of each pixel on the reconstruction plane based on said back-projected line data; and determining back-projected data by adding the back-projected pixel data on a pixel-by-pixel basis for all views used in image reconstruction.

According to the CT image producing method of the seventh aspect, since the three-dimensional image reconstruction technique is performed as proposed in Japanese Patent Application Nos. 2002-147231 and 2002-338947, the volume of calculation can be significantly reduced.

In its eighth aspect, the present invention provides the CT image producing method having the aforementioned configuration, characterized in that: representing a direction perpendicular to a plane of rotation of the X-ray tube and multi-row detector or a direction of rectilinear motion of a helical scan as a z-direction, a direction of the center axis of the X-ray beam at a view angle *view* = 0° as a y-direction, and a direction orthogonal to the z- and y-directions as an x-direction, the line direction is defined as the x-direction for -45° ≤ *view* < 45° or a view angle range mainly including the range and also including its vicinity and 135° ≤ *view* < 225° or a view angle range mainly including the range and also including its vicinity, and the line direction is defined as the y-direction for 45° ≤ *view* < 135° or a view angle range mainly including the range and also including its vicinity and 225° ≤ *view* < 315° or a view angle range mainly including the range and also including its vicinity.

As used herein, *view* = -45° and *view* = 315° are actually equal and represent the same view angle, although they are differently denoted for convenience of expression.

When a line on a reconstruction plane is projected in the direction of X-ray transmission, accuracy increases for an angle between the line and direction of X-ray transmission closer to 90°, and decreases for an angle closer to 0°.

According to the CT image producing method of the eighth aspect, since the angle between the line and direction of X-ray transmission is no less than about 45°, accuracy reduction can be prevented.

In its ninth aspect, the present invention provides the CT image producing method having the aforementioned configuration, characterized in that: the subject to be imaged is a heart, and the phase of the cyclic motion is detected based on electrocardiographic signals.

According to the CT image producing method of the ninth aspect, a CT image of the heart at a desired phase can be suitably produced.

In its tenth aspect, the present invention provides an X-ray CT apparatus characterized in comprising: an X-ray tube; a multi-row detector; scanning means for scanning a subject to be imaged over a plurality of cycles while rotating at least one of said X-ray tube and multi-row detector around said subject to be imaged; and image reconstructing means for extracting projection data collected in the same phase range in said plurality of cycles, said projection data being collected at a projection point onto which a pixel on a reconstruction plane is projected on a multi-row detector plane in a direction of X-ray transmission, and producing a CT image based on said extracted projection data.

According to the X-ray CT apparatus of the tenth aspect, the CT image producing method of the first aspect can be suitably implemented.

In its eleventh aspect, the present invention provides an X-ray CT apparatus characterized in comprising: an X-ray tube; a multi-row detector; scanning means for scanning a subject to be imaged over a plurality of cycles while rotating at least one of said X-ray tube and multi-row detector around said subject to be imaged and making relative rectilinear motion of said X-ray tube and multi-row detector with respect to said subject to be imaged; and image reconstructing means for extracting projection data collected in the same phase range in said plurality of cycles, said projection data being collected at a projection point onto which a pixel on a reconstruction plane is projected on a multi-row detector plane in a direction of X-ray transmission, and producing a CT image based on said extracted projection data.

According to the X-ray CT apparatus of the eleventh aspect, the CT image producing method of the second aspect can be suitably implemented.

In its twelfth aspect, the present invention provides the X-ray CT apparatus having the aforementioned configuration, characterized in that: when a view range V needed in image reconstruction is divided into N segments from first through N-th segments (N ≥ 2), said scanning means repeats N times the collection of data for one segment in one cycle.

According to the X-ray CT apparatus of the twelfth aspect, the CT image producing method of the third aspect can be suitably implemented.

In its thirteenth aspect, the present invention provides the X-ray CT apparatus having the aforementioned configuration, characterized in that: representing the cycle of motion of the subject to be imaged as T, the time width of said phase range as $\tau e$, and the cycle of rotation of the X-ray tube and multi-row detector as Ts, said scanning means defines:

$$Ts = T - \tau e$$

or

$$Ts = T + \tau e.$$

According to the X-ray CT apparatus of the thirteenth aspect, the CT image producing method of the fourth aspect can be suitably implemented.

In its fourteenth aspect, the present invention provides the X-ray CT apparatus having the aforementioned configuration, characterized in that: the view range V needed in image reconstruction is V = (180° + fan beam angle).

According to the X-ray CT apparatus of the fourteenth aspect, the CT image producing method of the fifth aspect can be suitably implemented.

In its fifteenth aspect, the present invention provides the X-ray CT apparatus having the aforementioned configuration, characterized in that: said image reconstructing means produces a CT image by a three-dimensional image reconstruction technique.

According to the X-ray CT apparatus of the fifteenth aspect, the CT image producing method of the sixth aspect can be suitably implemented.

In its sixteenth aspect, the present invention provides the X-ray CT apparatus having the aforementioned configuration, characterized in that said three-dimensional image reconstruction technique is a three-dimensional back-projection method comprising: extracting projection data corresponding to a projection line formed by projecting one line or a plurality of parallel lines at spacings of a plurality of pixels on a reconstruction plane onto a multi-row detector plane in a direction of X-ray transmission; generating projection line data by multiplying said extracted projection data by a cone beam reconstruction weight; generating back-projected line data by filtering said projection line data; determining back-projected pixel data of each pixel on the reconstruction plane based on said back-projected line data; and determining back-projected data by adding the back-projected pixel data on a pixel-by-pixel basis for all views used in image reconstruction.

According to the X-ray CT apparatus of the sixteenth aspect, the CT image producing method of the seventh aspect can be suitably implemented.

In its seventeenth aspect, the present invention provides the X-ray CT apparatus having the aforementioned configuration, characterized in that: representing a direction perpendicular to a plane of rotation of the X-ray tube and multi-row detector or a direction of rectilinear motion of a helical scan as a z-direction, a direction of the center axis of the X-ray beam at a view angle *view* = 0° as a y-direction, and a direction orthogonal to the z- and y-directions as an x-direction, the line direction is defined as the x-direction for -45° ≤ *view* < 45° or a view angle range mainly including the range and also including its vicinity and 135° ≤ *view* < 225° or a view angle range mainly including the range and also including its vicinity, and the line direction is defined as the y-direction for 45° ≤ *view* < 135° or a view angle range mainly including the range and also including its vicinity and 225° ≤ *view* < 315° or a view angle range mainly including the range and also including its vicinity.

According to the X-ray CT apparatus of the seventeenth aspect, the CT image producing method of the eighth aspect can be suitably implemented.

In its eighteenth aspect, the present invention provides the X-ray CT apparatus having the aforementioned configuration, characterized in further comprising: an electrocardiograph for detecting the phase of cyclic motion of a heart.

According to the X-ray CT apparatus of the eighteenth aspect, the CT image producing method of the ninth aspect can be suitably implemented.

According to the CT image producing method and X-ray CT apparatus of the present invention, a periodically moving subject to be imaged can be scanned using a multi-row detector to produce a CT image at a desired phase with reduced cone-beam artifacts.

The CT image producing method and X-ray CT apparatus of the present invention are applicable for producing a CT image of the heart at a desired phase.

The invention will now be described in greater detail, by way of example, with reference to the drawings, in which:-

Figure 1 is a block diagram showing the configuration of an X-ray CT apparatus of Example 1.

Figure 2 is an explanatory diagram showing a rotation of an X-ray tube and a multi-row detector.

Figure 3 is an explanatory diagram showing a cone beam.

Figure 4 is a flow chart showing processing of a multi-sector reconstruction technique by the X-ray CT apparatus of Example 1.

Figure 5 is an explanatory diagram showing a format of collected projection data when storing them.

Figure 6 is an explanatory diagram showing a view range needed in image reconstruction divided by segmentation.

Figure 7 is an explanatory diagram showing timing of extracting projection data for each segment.

Figure 8 is a flow chart showing details of three-dimensional image reconstruction processing.

Figure 9 is a conceptual diagram showing lines on a reconstruction plane P projected in the direction of X-ray transmission.

Figure 10 is a conceptual diagram showing lines on the reconstruction plane P projected onto a detector plane.

Figure 11 is a conceptual diagram showing projection data Dr on lines on the detector plane at a view angle *view* = 0° projected onto a projection plane.

Figure 12 is a conceptual diagram showing projection line data Dp obtained by multiplying the projection data Dr on the projection plane *pp* at the view angle *view* = 0° by a cone beam reconstruction weight.

Figure 13 is a conceptual diagram showing backprojected line data Df obtained by filtering the projection line data Dp on the projection plane *pp* at the view angle *view* = 0°.

Figure 14 is a conceptual diagram showing high density backprojected line data Dh obtained by interpolating the backprojected line data Df on the projection plane *pp* at the view angle *view* = 0°.

Figure 15 is a conceptual diagram showing backprojected pixel data D2 obtained by developing the high density backprojected line data Dh on the projection plane *pp* at the view angle *view* = 0° over lines on the reconstruction plane.

Figure 16 is a conceptual diagram showing backprojected pixel data D2 obtained by developing the high density backprojected line data Dh on the projection plane *pp* at the view angle *view* = 0° in between the lines on the reconstruction plane.

Figure 17 is a conceptual diagram showing projection data Dr on lines on the detector plane at a view angle *view* = 90° projected onto a projection plane.

Figure 18 is a conceptual diagram showing projection line data Dp obtained by multiplying the projection data Dr on the projection plane *pp* at the view angle *view* = 90° by a cone beam reconstruction weight.

Figure 19 is a conceptual diagram showing backprojected line data Df obtained by filtering the projection line data Dp on the projection plane *pp* at the view angle *view* = 90°.

Figure 20 is a conceptual diagram showing high density backprojected line data Dh obtained by interpolating the backprojected line data Df on the projection plane *pp* at the view angle *view* = 90°.

Figure 21 is a conceptual diagram showing backprojected pixel data D2 obtained by developing the high density backprojected line data Dh on the projection plane *pp* at the view angle *view* = 90° over lines on the reconstruction plane.

Figure 22 is a conceptual diagram showing backprojected pixel data D2 obtained by developing the high density backprojected line data Dh on the projection plane *pp* at the view angle *view* = 90° in between the lines on the reconstruction plane.

Figure 23 is an explanatory diagram showing back-projected data D3 obtained by adding the backprojected pixel data D2 on a pixel-by-pixel basis for all views.

The present invention will now be described in more detail with reference to embodiments shown in the accompanying drawings. It should be noted that the present invention is not limited to the embodiments.

[Example 1]

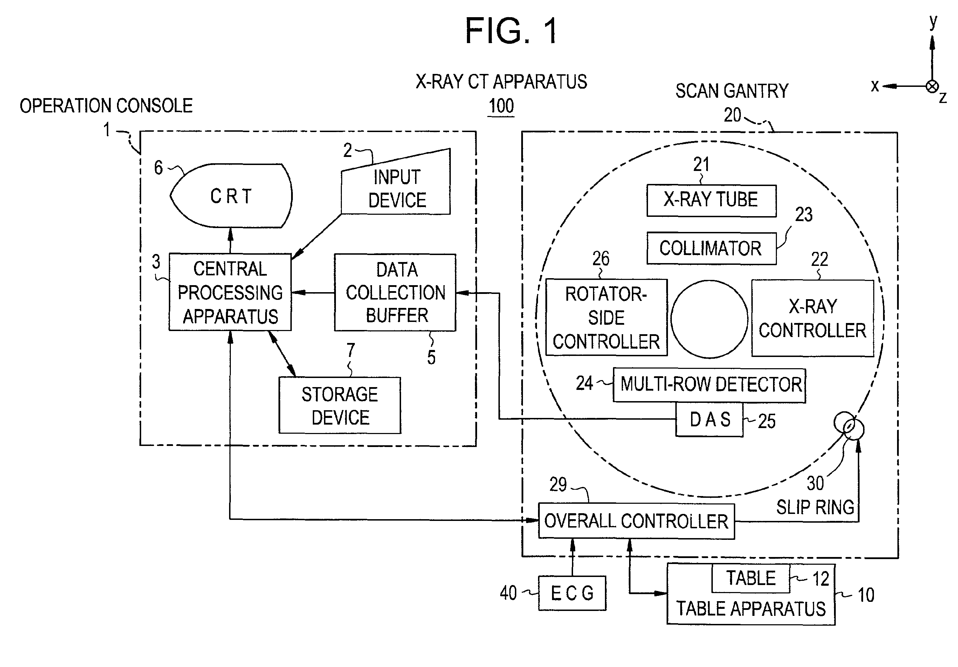

Figure 1 is a block diagram showing the configuration of an X-ray CT apparatus 100 of Example 1.

The X-ray CT apparatus 100 comprises an operation console 1, a table apparatus 10, a scan gantry 20, and an electrocardiograph (ECG) 40.

The operation console 1 comprises an input device 2 for accepting inputs by a human operator, a central processing apparatus 3 for executing image reconstruction processing etc., a data collection buffer 5 for collecting projection data acquired at the scan gantry 20, a CRT 6 for displaying a CT image reconstructed from the projection data, and a storage device 7 for storing programs, data, and X-ray CT images.

The table apparatus 10 comprises a table 12 for laying thereon a subject to be imaged and transporting the subject into/out of a bore (cavity portion) of the scan gantry 20. The table 12 is vertically moved and rectilinearly moved by a motor incorporated in the table apparatus 10.

The scan gantry 20 comprises an X-ray tube 21, an X-ray controller 22, a collimator 23, a multi-row detector 24, a DAS (data acquisition system) 25, a rotator-side controller 26 for controlling the X-ray controller 22, collimator 23 and DAS 25, an overall controller 29 for communicating control signals etc. with the operation console 1 and imaging table 10, and a slip ring 30.

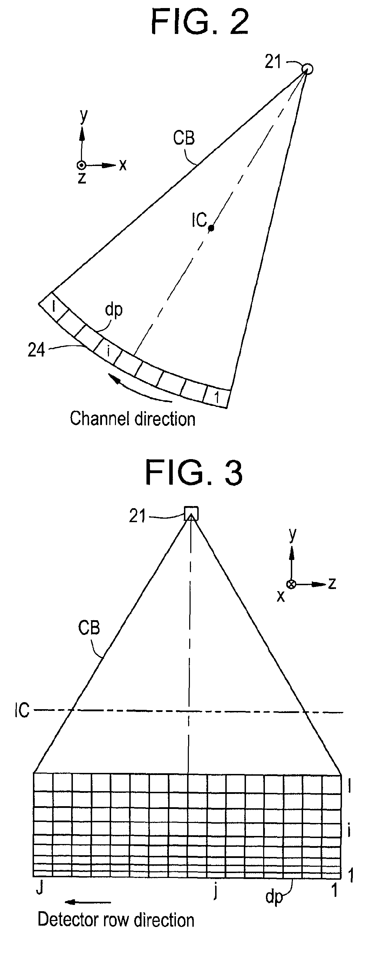

Figures 2 and 3 are explanatory diagrams of the X-ray tube 21 and multi-row detector 24.

The X-ray tube 21 and multi-row detector 24 rotate around a center of rotation IC. Representing the direction of rectilinear motion of the table 12 as a z-direction, a direction perpendicular to the upper surface of the table 12 as a y-direction, and a direction orthogonal to the z- and y-directions as an x-direction, a plane of rotation of the X-ray tube 21 and multi-row detector 24 is an x-y plane.

The X-ray tube 21 generates an X-ray beam CB generally referred to as a cone beam. When the direction of the center axis of the X-ray beam CB is parallel to the y-direction, a view angle $view = 0°$ is defined.

The multi-row detector 24 has J (e.g., J = 256) detector rows. Each detector row has I (e.g., I = 1,024) channels.

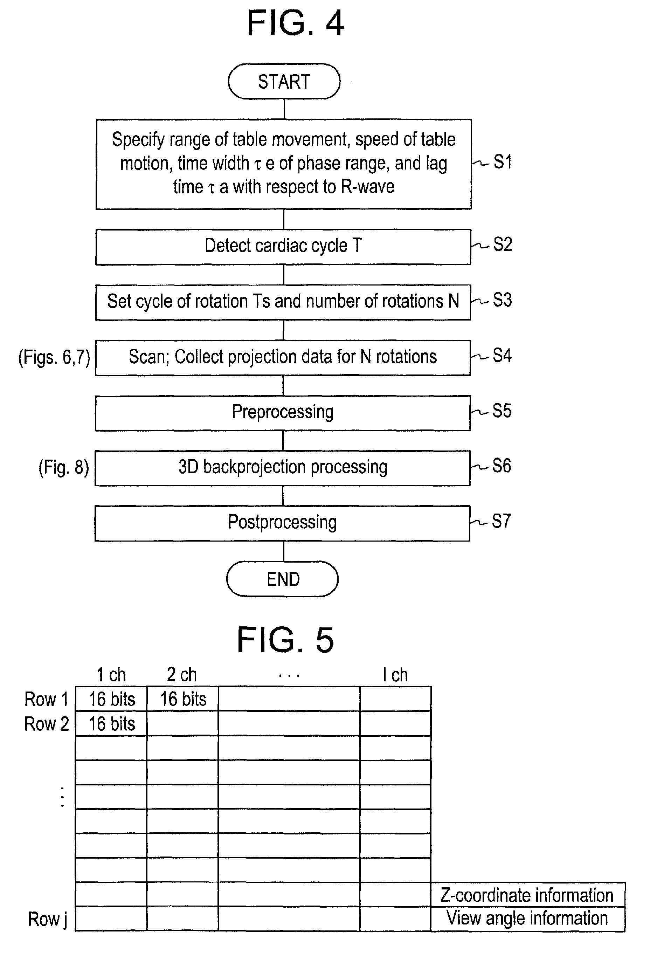

Figure 4 is a flow chart showing processing of the multi-sector reconstruction technique using the X-ray CT apparatus 100.

At Step S1, the operator inputs via the input device 2 a range of table movement, a speed of table motion, a time width $\tau e$ of a desired phase range, and a lag time $\tau a$ with respect to an R-wave.

At Step S2, the central processing apparatus 3 detects a cardiac cycle T by the electrocardiograph 40.

At Step S3, the central processing apparatus 3 calculates and sets the cycle of rotation Ts and the number of rotations N (= the number of divided segments) of the X-ray tube 21 and multi-row detector 24 according to the following equation. The view range needed in image reconstruction is represented as $V°$ (degrees):

$$Ts = T - \tau e,$$

and

$$N = (V / 360°) \times T / \tau e.$$

If T = 1 sec., $\tau e$ = 1 / 6 sec., and V = 240°, for example, then Ts = 5 / 6 sec., and N = 4.

At Step S4, while rotating the X-ray tube 21 and multi-row detector 24 around the heart (= subject to be imaged) and rectilinearly moving the table 12, projection data D0($z$, $view$, $j$, $i$) represented by the rectilinear motion position $z$, view angle $view$, detector row index $j$ and channel index $i$ are collected for N rotations. The rectilinear motion position $z$ is obtained by an encoder counting a z-position pulse, converted into a z-coordinate at the overall controller 29, passed via the slip ring 30, and appended as z-coordinate information to the projection data from the DAS 25.

Figure 5 shows a format of the projection data at a certain view appended with the z-coordinate information.

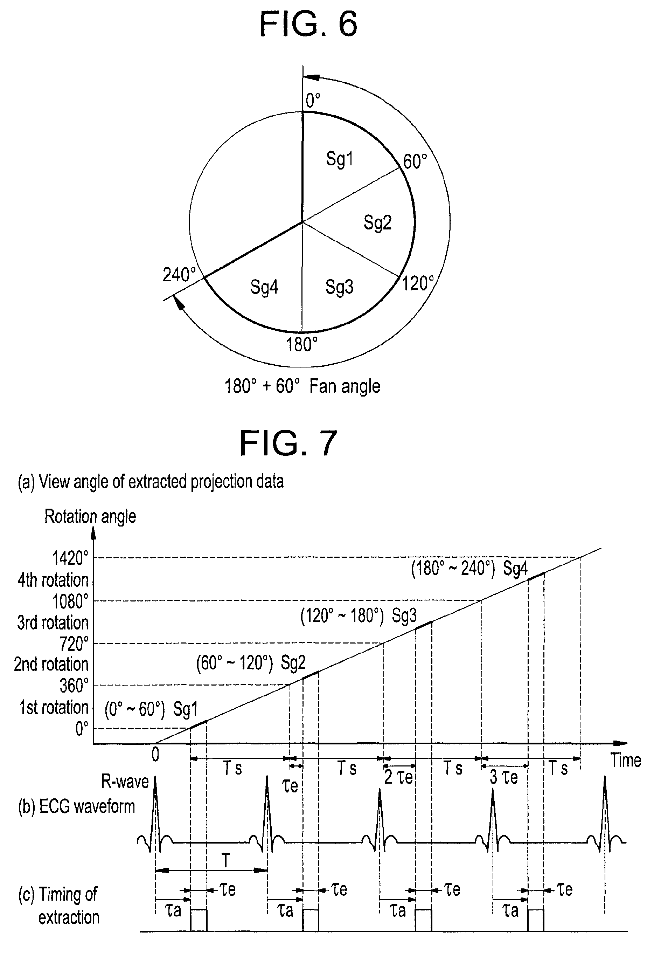

The data collection processing at Step S4 will be discussed later with reference to Figures 6 and 7.

At Step S5, the projection data D0($z$, $view$, $j$, $i$) is subjected to pre-processing (offset correction, log correction, X-ray dose correction and sensitivity correction).

At Step S6, the pre-processed projection data D0($z$, $view$, $j$, $i$) is subjected to three-dimensional back-projection processing to determine back-projected data D3($x$, $y$).

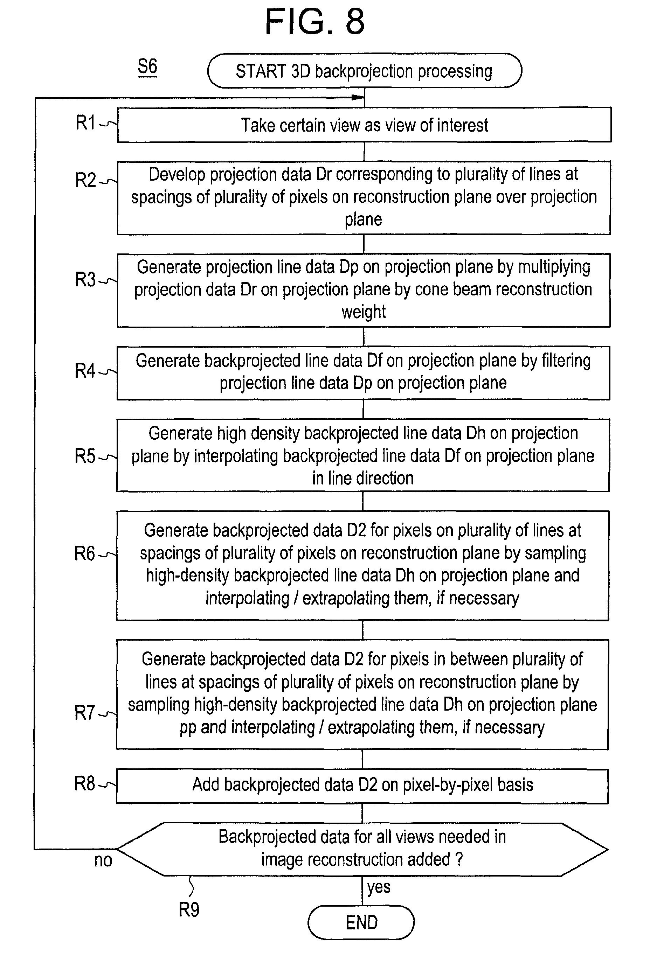

The three-dimensional back-projection processing at Step S6 will be discussed later with reference to Figure 8.

At Step S7, the back-projected data D3($x$, $y$) is subjected to post-processing to obtain a CT image.

Referring to Figure 6, the multi-sector reconstruction technique divides a view range needed in image reconstruction into N segments from first through N-th segments (N ≥ 2). In Figure 6, the view range V needed in image reconstruction is V = (180° + fan angle), fan angle = 60°, and N = 4.

Referring to Figure 7, if projection data at a view angle at a time delayed by τa with reference to an R-wave among projection data collected during a first rotation is defined as projection data at *view* = 0°, a data set over a view range needed in image reconstruction can be generated by extracting projection data in a view angle range ((n - 1) × 360° × τe / T) — (n × 360° × τe / T) from projection data collected in an *n*-th (*n* = 1 — N) rotation. The projection data in that data set all represent a phase range having a time width τe from the time delayed by τa with reference to an R-wave.

Figure 8 is a flow chart showing details of the three-dimensional backprojection processing (Step S6 in Figure 4).

At Step R1, one view is taken as a view of interest from a data set obtained at Step S4 of Figure 4. At Step R2, projection data Dr corresponding to a plurality of parallel lines at spacings of a plurality of pixels on a reconstruction plane P are extracted from the projection data D0($z$, *view*, $j, i$) at the view of interest.

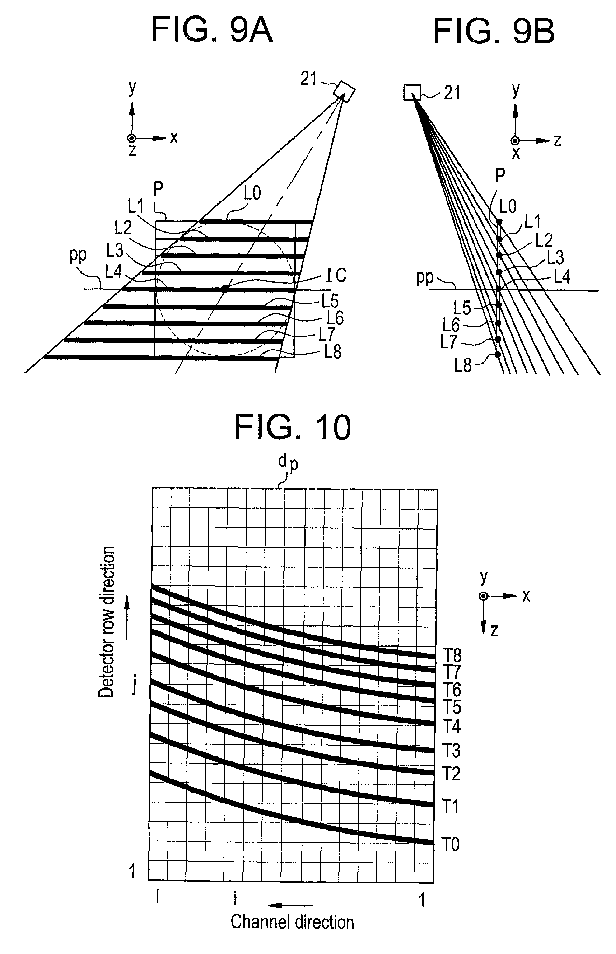

Figure 9 exemplarily shows a plurality of parallel lines L0 — L8 on the reconstruction plane P.

The number of lines is 1/64 — 1/2 of the maximum number of pixels in the reconstruction plane in a direction orthogonal to the lines. For example, if the number of pixels in the reconstruction plane P is 512×512, the number of lines is nine.

Moreover, the line direction is defined as the x-direction for -45° ≤ *view* < 45° (or a view angle range mainly including the range and also including its vicinity) and 135° ≤ *view* < 225° (or a view angle range mainly including the range and also including its vicinity). The line direction is defined as the y-direction for 45° ≤ *view* < 135° (or a view angle range mainly including the range and also including its vicinity) and 225° ≤ *view* < 315° (or a view angle range mainly including the range and also including its vicinity).

Furthermore, a projection plane *pp* is assumed to pass through the center of rotation IC and be parallel to the lines L0 — L8.

Figure 10 shows lines T0 — T8 formed by projecting the plurality of parallel lines L0 — L8 on the reconstruction plane P onto a detector plane *dp* in a direction of X-ray transmission.

The direction of X-ray transmission is determined depending upon the geometry of the X-ray tube 21, multi-row detector 24 and lines L0 — L8.

The projection data Dr corresponding to the lines L0 — L8 can be obtained by extracting projection data at the detector row $j$ and channel $i$ corresponding to the lines T0 — T8 projected onto the detector plane *dp.*

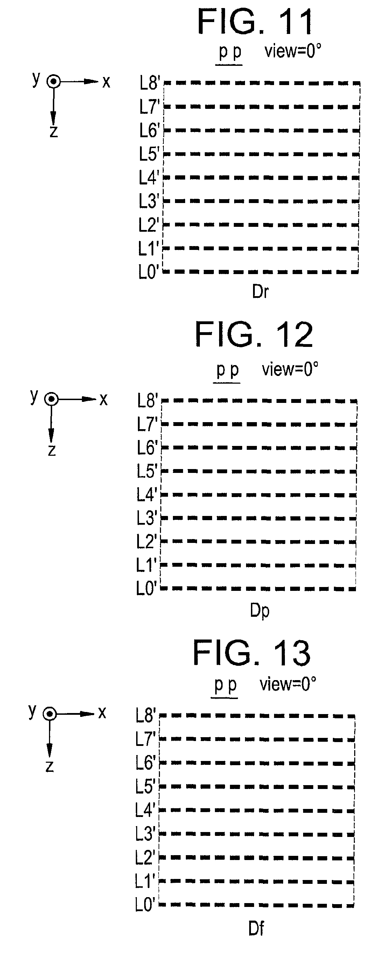

Now lines L0' — L8' formed by projecting the lines T0 — T8 onto the projection plane *pp* in the direction of X-ray transmission are assumed as shown in Figure 11, and the projection data Dr are developed over the lines L0' — L8' on the projection plane *pp*.

Referring again to Figure 8, at Step R3, the projection data Dr of the lines L0' — L8' on the projection plane *pp* are multiplied by a cone beam reconstruction weight to generate projection line data Dp on the projection plane *pp* as shown in Figure 12.

The cone beam reconstruction weight is $(r1/r0)^2$, where $r0$ is the distance from the focal spot of the X-ray tube 21 to the $j$-th detector row and $i$-th channel of the multi-row detector 24 corresponding to projection data Dr, and $r1$ is the distance from the focal spot of the X-ray tube 21 to a point on the reconstruction plane P corresponding to the projection data Dr.

At Step R4, the projection line data Dp on the projection plane *pp* are filtered. Specifically, the projection line data Dp on the projection plane *pp* are subjected to FFT, multiplied by a filter function (reconstruction function), and subjected to inverse FFT to generate image back-projected line data Df on the projection plane *pp* as shown in Figure 13.

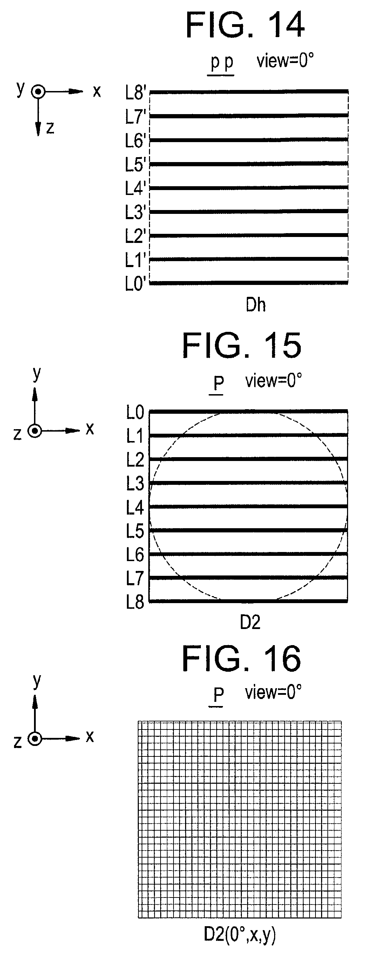

At Step R5, the back-projected line data Df on the projection plane *pp* is interpolated in the line direction to generate high-density back-projected line data Dh on the projection plane *pp* as shown in Figure 14.

The data density of the high-density back-projected line data Dh on the projection plane *pp* is 8 — 32 times the maximum number of pixels in the reconstruction plane P in the line direction. For example, if the factor is 16 and the number of pixels in the reconstruction plane P is 512×512, the data density is 8,192 points/line.

At Step R6, the high-density back-projected line data Dh on the projection plane pp are sampled and interpolated/extrapolated, if necessary, to generate back-projected pixel data D2 for pixels on the lines L0 — L8 on the reconstruction plane P, as shown in Figure 15.

At Step R7, the high-density back-projected line data Dh are sampled and interpolated/extrapolated to generate back-projection data D2 for pixels in between the lines L0 — L8, as shown in Figure 16. Alternatively, the interpolation/extrapolation is conducted based on the back-projected pixel data D2 for pixels on the lines L0 — L8 on the reconstruction plane P to generate back-projected pixel data D2 for pixels in between the lines L0 - L8.

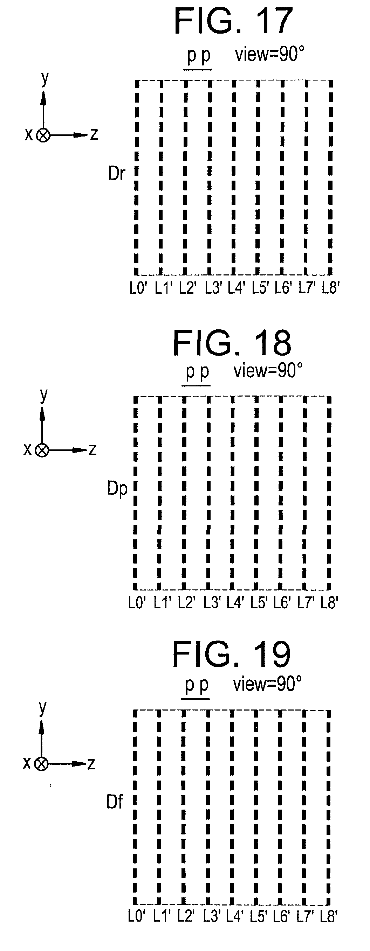

In Figures 11 — 16, -45° ≤ *view* < 45° (or a view angle range mainly including the range and also including its vicinity) and 135° ≤ *view* < 225° (or a view angle range mainly including the range and also including its vicinity) are assumed, while Figures 17 — 22 are applied for 45° ≤ *view* < 135° (or a view angle range mainly including the range and also including its vicinity) and 225° ≤ *view* < 315° (or a view angle range mainly including the range and also including its vicinity).

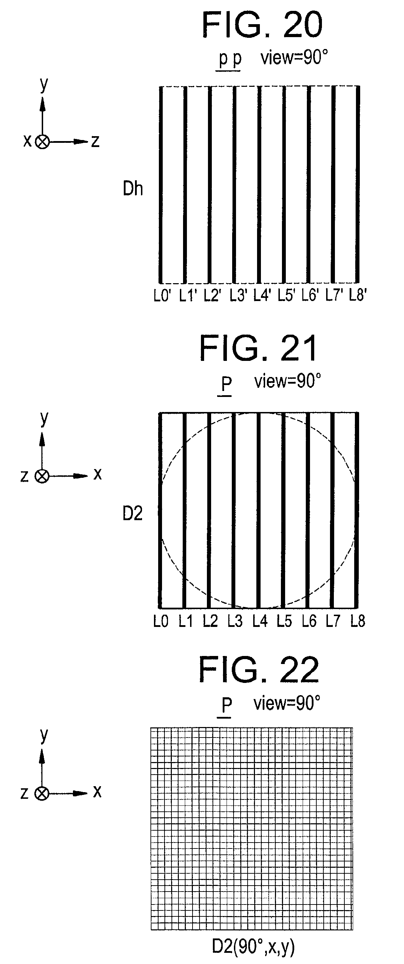

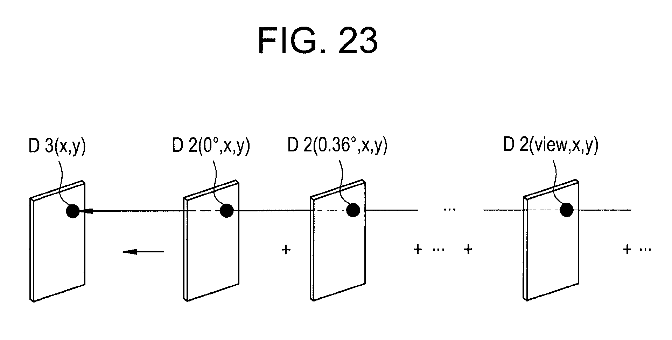

Referring again to Figure 8, at Step R8, the back-projected pixel data D2 shown in Figure 16 or 22 are added on a pixel-by-pixel basis, as shown in Figure 23.

At Step R9, Steps R1 — R8 are repeated for all views in the data set obtained at Step S4 of Figure 4 to obtain back-projected data D3$(x, y)$.

According to the X-ray CT apparatus 100 of Example 1, since the multi-sector reconstruction technique is employed, a CT image can be produced at a desired cardiac phase. Moreover, since the CT image is produced by extracting projection data of a detector row j and channel i upon which an X-ray beam passing through a pixel on a reconstruction plane P impinges, cone-beam artifacts can be reduced.

[Example 2]

While in Example 1, only projection data corresponding to the lines L0 — L8 on the reconstruction plane P are extracted from the data set, projection data corresponding to all pixels on the reconstruction plane P may be extracted to reconstruct a CT image.

[Example 3]

The technique for image reconstruction may be a conventionally known three-dimensional image reconstruction technique according to the Feldkamp method. Moreover, three-dimensional image reconstruction techniques proposed in Japanese Patent Application Nos. 2002-066420, 2002-147061, 2002-147231, 2002-235561, 2002-235662, 2002-267833, 2002-322756 and 2002-338947 may be employed.

**Claims**

1.  A CT image producing method comprising the steps of: scanning a periodically moving subject to be imaged over a plurality of cycles while making relative rotation of at least one of an X-ray tube (21) and a multi-row detector (24) around said subject to be imaged; extracting projection data collected in the same phase range in said plurality of cycles, said projection data being collected at a projection point onto which a pixel on a reconstruction plane is projected on a multi-row detector plane in a direction of X-ray transmission; and producing a CT image based on said extracted projection data.

2.  An X-ray CT apparatus (100) comprising: an X-ray tube (21); a multi-row detector (24); a scanning device (20) for scanning a subject to be imaged over a plurality of cycles while making relative rotation of at least one of said X-ray tube and multi-row detector around said subject to be imaged; and an image reconstructing device (3) for extracting projection data collected in the same phase range in said plurality of cycles, said projection data being collected at a projection point onto which a pixel on a reconstruction plane is projected on a multi-row detector plane in a direction of X-ray transmission, and producing a CT image based on said extracted projection data.

3.  An X-ray CT apparatus (100) comprising: an X-ray tube (21); a multi-row detector (24); a scanning device (20) for scanning a subject to be imaged over a plurality of cycles while making relative rotation of at least one of said X-ray tube and multi-row detector around said subject to be imaged and making relative rectilinear motion of said X-ray tube and multi-row detector with respect to said subject to be imaged; and an image reconstructing device (3) for extracting projection data collected in the same phase range in said plurality of cycles, said projection data being collected at a projection point onto which a pixel on a reconstruction plane is projected on a multi-row detector plane in a direction of X-ray transmission, and producing a CT image based on said extracted projection data.

4.  The X-ray CT apparatus (100) of claim 2 or 3, wherein: when a view range V needed in image reconstruction is divided into N segments from first through N-th segments (N $\geq$ 2), said scanning device repeats N times the collection of data for one segment in one cycle.

5.  The X-ray CT apparatus (100) of claim 4, wherein: representing the cycle of motion of the subject to be imaged as T, the time width of said phase range as $\tau$e, and the cycle of rotation of the X-ray tube (21) and multi-row detector (24) as Ts, said scanning device (20) defines:

$$Ts = T - \tau e$$

or

$$Ts = T + \tau e.$$

6.  The X-ray CT apparatus (100) of any one of claim 2 — 5, wherein: the view range V needed in image reconstruction is V = (180° + fan beam angle).

7.  The X-ray CT apparatus (100) of any one of claims 2 — 6, wherein: said image reconstructing device (3) produces a CT image by a three-dimensional image reconstruction technique.

8.  The X-ray CT apparatus (100) of claim 7, wherein said three-dimensional image reconstruction technique is a three-dimensional back-projection method comprising: extracting projection data corresponding to a projection line formed by projecting one line or a plurality of parallel lines at spacings of a plurality of pixels on a reconstruction plane onto a multi-row detector plane in a direction of X-ray transmission; generating projection line data by multiplying said extracted projection data by a cone beam reconstruction weight; generating back-projected line data by filtering said projection line data; determining back-projected pixel data of each pixel on the reconstruction field based on said back-projected line data; and determining back-projected data by adding the back-projected pixel data on a pixel-by-pixel basis for all views used in image reconstruction.

9.   The X-ray CT apparatus (100) of claim 8, wherein: representing a direction perpendicular to a plane of rotation of the X-ray tube and multi-row detector or a direction of rectilinear motion of a helical scan as a z-direction, a direction of the center axis of the X-ray beam at a view angle $view = 0°$ as a y-direction, and a direction orthogonal to the z- and y-directions as an x-direction, the line direction is defined as the x-direction for $-45° \leq view < 45°$ or a view angle range mainly including the range and also including its vicinity and $135° \leq view < 225°$ or a view angle range mainly including the range and also including its vicinity, and the line direction is defined as the y-direction for $45° \leq view < 135°$ or a view angle range mainly including the range and also including its vicinity and $225° \leq view < 315°$ or a view angle range mainly including the range and also including its vicinity.

10.  The X-ray CT apparatus (100) of any one of claims 2 — 9, further comprising: an electrocardiograph (40) for detecting the phase of cyclic motion of a heart.

# FIG. 1

X-RAY CT APPARATUS
100

OPERATION CONSOLE 1

SCAN GANTRY 20

6 — CRT

2 — INPUT DEVICE

3 — CENTRAL PROCESSING APPARATUS

DATA COLLECTION BUFFER 5

STORAGE DEVICE 7

21 — X-RAY TUBE

COLLIMATOR 23

26 — ROTATOR-SIDE CONTROLLER

X-RAY CONTROLLER 22

24 — MULTI-ROW DETECTOR

DAS 25

30 SLIP RING

29 — OVERALL CONTROLLER

40 — ECG

TABLE 12

TABLE APPARATUS 10

EP 1 530 163 A2

# FIG. 2

Channel direction

# FIG. 3

Detector row direction

# FIG. 4

```
            ( START )
                |
S1 — Specify range of table movement, speed of table
     motion, time width τe of phase range, and lag
     time τa with respect to R-wave
                |
S2 — Detect cardiac cycle T
                |
S3 — Set cycle of rotation Ts and number of rotations N
                |
(Figs. 6,7)  S4 — Scan; Collect projection data for N rotations
                |
S5 — Preprocessing
                |
(Fig. 8)  S6 — 3D backprojection processing
                |
S7 — Postprocessing
                |
            ( END )
```

# FIG. 5

| | 1 ch | 2 ch | · · · | I ch | |
|---|---|---|---|---|---|
| Row 1 | 16 bits | 16 bits | | | |
| Row 2 | 16 bits | | | | |
| | | | | | |
| | | | | | |
| ⋮ | | | | | |
| | | | | | |
| | | | | | |
| | | | | | |
| | | | | | Z-coordinate information |
| Row j | | | | | View angle information |

# FIG. 6

180° + 60°  Fan angle

# FIG. 7

(a) View angle of extracted projection data

# FIG. 8

S6  ( START 3D backprojection processing )

R1 — | Take certain view as view of interest |

R2 — | Develop projection data Dr corresponding to plurality of lines at spacings of plurality of pixels on reconstruction plane over projection plane |

R3 — | Generate projection line data Dp on projection plane by multiplying projection data Dr on projection plane by cone beam reconstruction weight |

R4 — | Generate backprojected line data Df on projection plane by filtering projection line data Dp on projection plane |

R5 — | Generate high density backprojected line data Dh on projection plane by interpolating backprojected line data Df on projection plane in line direction |

R6 — | Generate backprojected data D2 for pixels on plurality of lines at spacings of plurality of pixels on reconstruction plane by sampling high-density backprojected line data Dh on projection plane and interpolating / extrapolating them, if necessary |

R7 — | Generate backprojected data D2 for pixels in between plurality of lines at spacings of plurality of pixels on reconstruction plane by sampling high-density backprojected line data Dh on projection plane pp and interpolating / extrapolating them, if necessary |

R8 — | Add backprojected data D2 on pixel-by-pixel basis |

no     < Backprojected data for all views needed in image reconstruction added ? >

R9

yes

( END )

# FIG. 9A

# FIG. 9B

# FIG. 10

# FIG. 11

p p    view=0°

Dr

# FIG. 12

p p    view=0°

Dp

# FIG. 13

p p    view=0°

Df

# FIG. 14

p p    view=0°

Dh

# FIG. 15

P    view=0°

D2

# FIG. 16

P    view=0°

D2(0°,x,y)

# FIG. 17

# FIG. 18

# FIG. 19

# FIG. 20

p p   view=90°

y

x ⊗ ──→ z

Dh

L0' L1' L2' L3' L4' L5' L6' L7' L8'

# FIG. 21

P   view=90°

y

z ⊗ ──→ x

D2

L0 L1 L2 L3 L4 L5 L6 L7 L8

# FIG. 22

P   view=90°

y

z ⊗ ──→ x

D2(90°,x,y)

# FIG. 23

D 3(x,y)    D 2(0°,x,y)    D 2(0.36°,x,y)         D 2(view,x,y)